# FASCICULE DE BREVET EUROPEEN

(11) **EP 1 000 153 B1**
(45) Date de publication et mention de la délivrance du brevet: **27.10.2004**
(21) Numéro de dépôt: 98939715.3
(22) Date de dépôt: 20.07.1998
(51) Int. Cl.: C12N 15/12, C07K 14/435, A61K 38/17

(54) **PEPTIDES ANTI-MICROBIENS DE CRUSTACES, DENOMMES PENAEIDINES**
PENAEIDINE: ANTIMIKROBIELLE PEPTIDE AUS KREBSTIEREN
CRUSTACEAN ANTIMICROBIAL PEPTIDES

(30) Priorité: 21.07.1997 FR 9709214
(43) Date de publication de la demande: 17.05.2000
(73) Titulaire: Institut Français de Recherche pour l'Exploitation de la Mer (IFREMER), 92138 Issy-les-Moulineaux (FR); CENTRE NATIONAL DE LA RECHERCHE SCIENTIFIQUE (CNRS), 75794 Paris Cedex 16 (FR)
(72) Inventeur: DESTOUMIEUX, Delphine, F-34070 Montpellier (FR); BACHERE, Evelyne, F-34830 Clapiers (FR); BULET, Philippe, F-67550 Vendenheim (FR)
(74) Mandataire: Vialle-Presles, Marie José
(86) Numéro de dépôt international: PCT/FR1998/001583
(87) Numéro de publication internationale: WO 1999/005270

(56) Documents cités:
- DESTOUMIEUX D ET AL: "Penaeidins, a new family of antimicrobial peptides isolated from the shrimp Penaeus vannamei (Decapoda)." J BIOL CHEM, NOV 7 1997, 272 (45) P28398-406, XP002060968 UNITED STATES
- SCHNAPP D ET AL: "Purification and characterization of a proline-rich antibacterial petide, with sequence similarity to bactenecin-7, from the haemocytes of the shore crab, Carcinus maenas" EUR. JOURNAL OF BIOCHEMISTRY, vol. 3, 1996, pages 532-539, XP002060970 cité dans la demande
- EHRET-SABATIER L ET AL: "Characterization of novel cystein-rich antimicrobial peptides from scorpion blood" JOURNAL OF BIOLOGICAL CHEMISTRY., vol. 271, 1996, pages 29537-29544, XP002060972 MD US cité dans la demande
- KUHN R ET AL:'Male specific sperm protein MST84DC' Swissprot Database entry M84c_Drome Accession number Q01644, 01-Jul-1993 XP002091985

### LISTAGE DE SEQUENCES

<110> DESTOUMIEUX, Delphine
   BACHERE, Evelyne
   BULET, Philippe
   IFREMER
   CNRS
<120> PEPTIDES ANTI-MICROBIENS DE CRUSTACES
<130> MJPrm712/5
<140>
   <141>
<150> FR9709214
   <151> 1997-07-21
<160> 13
<170> PatentIn Vers. 2.0
<210> 1
   <211> 658
   <212> ADN
   <213> Penaeus vannamei
<220>
   <221> CDS
   <222> (76)..(294)
<220>
   <221> mat_peptide
   <222> (139)..(294)
<400> 1
   aattcggcac gagctccctc tagcctcacc tgcagagacc gacgctccga gcccgggttc 60
   cctcctgcgt ccgcc atg cgc ctc gtg gtc tgc ctg gtc ttc ttg gcc tcc  111
                    Met Arg Leu Val Val Cys Leu Val Phe Leu Ala Ser
                        -20                 -15                 -10
   ttc gcc ctg gtc tgc caa ggc gaa gcg tac agg ggc ggt tac aca ggc   159
   Phe Ala Leu Val Cys Gln Gly Glu Ala Tyr Arg Gly Gly Tyr Thr Gly
                   -5                  -1   1               5
   ccg ata ccc agg cca cca ccc att gga aga cca ccg ttc aga cct gtt   207
   Pro Ile Pro Arg Pro Pro Pro Ile Gly Arg Pro Pro Phe Arg Pro Val
           10                  15                  20
   tgc aat gca tgc tac aga ctt tcc gtc tca gat gct cgc aat tgc tgc   255
   Cys Asn Ala Cys Tyr Arg Leu Ser Val Ser Asp Ala Arg Asn Cys Cys
        25                  30                  35
   atc aag ttc gga agc tgt tgt cac tta gta aaa gga taa agaaattgac    304
   Ile Lys Phe Gly Ser Cys Cys His Leu Val Lys Gly
    40                  45                  50
   ggagaagaca atggaaacct ggcttgacaa cttgttaatt aatactcata tgtgaagaga 364
   ttgcaaccct gatttgtgtc aaggatgtgg gtatttcgtc tatccatcgc taaagattct 424
   tccatgaatg tatgatgaag gaaagtgcat gtgtgtaagt atgtatgtat gtgcttacag 484
   gtatttgttg cattaagtgt ccgtgtattt aggatctgca acacacgagg aagagaatat 544
   ttgccacttg ccatttattt cagtttctgt aagtgtggat ctgtgagagg ttggtgttga 604
   cagatctctc ttttacaaat aaatttgata tctgtgaaaa aaaaaaaaaa aaaa       658
<210> 2
   <211> 72
   <212> PRT
   <213> Penaeus vannamei
<400> 2
   Met Arg Leu Val Val Cys Leu Val Phe Leu Ala Ser Phe Ala Leu Val
    1               5                  10                  15
   Cys Gln Gly Glu Ala Tyr Arg Gly Gly Tyr Thr Gly Pro Ile Pro Arg
               20                  25                  30
   Pro Pro Pro Ile Gly Arg Pro Pro Phe Arg Pro Val Cys Asn Ala Cys
           35                  40                  45
   Tyr Arg Leu Ser Val Ser Asp Ala Arg Asn Cys Cys Ile Lys Phe Gly
       50                  55                  60
   Ser Cys Cys His Leu Val Lys Gly
   65                  70
<210> 3
   <211> 736
   <212> ADN
   <213> Penaeus vannamei
<220>
   <221> CDS
   <222> (71)..(319)
<220>
   <221> mat_peptide
   <222> (128)..(319)
<400> 3
   aattcggcac gagtcgagcc tcacctgcag agaccgacgc tccgagcccg ggttccctcc 60
   tgcgtccgcc atg cgc ctc gtg gtc tgc ctg gtc ttc ttg gcc tcc ttc   109
              Met Arg Leu Val Val Cys Leu Val Phe Leu Ala Ser Phe
                              -15                 -10
   gcc ctg gtc tgc caa ggc caa gtg tac aag ggc ggt tac acg cgc ccg   157
   Ala Leu Val Cys Gln Gly Gln Val Tyr Lys Gly Gly Tyr Thr Arg Pro
       -5              -1   1              5                  10
   ata ccc agg cca cca ccc ttc gtg aga cct ttg cca gga ggg cct att   205
   Ile Pro Arg Pro Pro Pro Phe Val Arg Pro Leu Pro Gly Gly Pro Ile
                   15                  20                  25
   ggt cca tac aac ggt tgc cct gtc tca tgc cgg gga att tcc ttc tca   253
   Gly Pro Tyr Asn Gly Cys Pro Val Ser Cys Arg Gly Ile Ser Phe Ser
               30                  35                  40
   caa gcg cgt tct tgc tgc tcc cgg tta ggg cgt tgc tgt cac gtg gga   301
   Gln Ala Arg Ser Cys Cys Ser Arg Leu Gly Arg Cys Cys His Val Gly
           45                  50                  55
   aag gga tat tcc ggt tga tggagaacac gatgaaaacc tcgcttgaca          349
   Lys Gly Tyr Ser Gly
       60
   acctgttgat tgatacttgt atgtgaagag actgtgatcc tgattttgca ctgtgttttc 409
   tcgttcaata ttcttactct ggcttgtgga atggatgtag ttatttgacc ctatgttttt 469
   ttttaagact tttccatgaa tgcacgatga atgaaagctt gcgtgatatg aatgagtgca 529
   tccacttttc aacgtcccag caggtggcgc cgtattcatg atttgtgaca cacgaggaag 589
   taaatccatg ccatctgcct ttcgttgtaa tttttagtga gtatggatct gtgtgtgggt 649
   gatttttaca aatctctcaa aggactttta gaaatgttac tcctttacaa ataaaattgg 709
   tatcttgaaa aaaaaaaaaa aaaaaaa                                     736
<210> 4
   <211> 82
   <212> PRT
   <213> Penaeus vannamei
<400> 4
   Met Arg Leu Val Val Cys Leu Val Phe Leu Ala Ser Phe Ala Leu Val
   1 5 10 15
   Cys Gln Gly Gln Val Tyr Lys Gly Gly Tyr Thr Arg Pro Ile Pro Arg
   20 25 30
   Pro Pro Pro Phe Val Arg Pro Leu Pro Gly Gly Pro Ile Gly Pro Tyr
   35 40 45
   Asn Gly Cys Pro Val Ser Cys Arg Gly Ile Ser Phe Ser Gln Ala Arg
   50 55 60
   Ser Cys Cys Ser Arg Leu Gly Arg Cys Cys His Val Gly Lys Gly Tyr
   65 70 75 80
   Ser Gly
<210> 5
   <211> 50
   <212> PRT
   <213> Penaeus vannamei
<400> 5
   Tyr Arg Gly Gly Tyr Thr Gly Pro Ile Pro Arg Pro Pro Pro Ile Gly
   1 5 10 15
   Arg Pro Pro Leu Arg Leu Val Val Cys Ala Cys Tyr Arg Leu Ser Val
   20 25 30
   Ser Asp Ala Arg Asn Cys Cys Ile Lys Phe Gly Ser Cys Cys His Leu
   35 40 45
   Val Lys
   50
<210> 6
   <211> 50
   <212> PRT
   <213> Penaeus vannamei
<400> 6
   Tyr Arg Gly Gly Tyr Thr Gly Pro Ile Pro Arg Pro Pro Pro Ile Gly
   1 5 10 15
   Arg Pro Pro Phe Arg Pro Val Cys Asn Ala Cys Tyr Arg Leu Ser Val
   20 25 30
   Ser Asp Ala Arg Asn Cys Cys Ile Lys Phe Gly Ser Cys Cys His Leu
   35 40 45
   Val Lys
   50
<210> 7
   <211> 62
   <212> PRT
   <213> Penaeus vannamei
<400> 7
   Gln Val Tyr Lys Gly Gly Tyr Thr Arg Pro Ile Pro Arg Pro Pro Pro
   1 5 10 15
   Phe Val Arg Pro Leu Pro Gly Gly Pro Ile Gly Pro Tyr Asn Gly Cys
   20 25 30
   Pro Val Ser Cys Arg Gly Ile Ser Phe Ser Gln Ala Arg Ser Cys Cys
   35 40 45
   Ser Arg Leu Gly Arg Cys Cys His Val Gly Lys Gly Tyr Ser
   50 55 60
<210> 8
   <211> 21
   <212> ADN
   <213> Penaeus vannamei
<220>
   <223> PCR primer
<220>
   <223> PCR primer
<400> 8
   gtgtacaagg gcggttacac g 21
<210> 9
   <211> 21
   <212> ADN
   <213> Penaeus vannamei
<220>
   <223> PCR primer
<400> 9
   caacaggttg tcaagcgagg t 21
<210> 10
   <211> 37
   <212> ADN
   <213> Artificial Sequence
<220>
   <223> PCR primer
<400> 10
   gcgcgcaagc ttggacaaga gatacagggg cggttac 37
<210> 11
   <211> 35
   <212> ADN
   <213> Artificial Sequence
<220>
   <223> PCR primer
<400> 11
<210> 12
   <211> 37
   <212> ADN
   <213> Artificial Sequence
<220>
   <223> PCR primer
<400> 12
   gcgcgcaagc ttggacaaga gacaagtgta caagggc 37
<210> 13
   <211> 28
   <212> ADN
   <213> Artificial Sequence
<220>
<220>
   <223> PCR primer
<400> 13
   gcgcgcggat cctcaaaccg gaatatcc                                 28

## Description

L'Invention est relative à de nouveaux peptides anti-microbiens produits par des crevettes pénéides.

Des peptides dotés de propriétés anti-microbiennes sont produits par une grande variété d'espèces (animales ou végétales), chez lesquelles ils participent à des mécanismes non-spécifiques de défense contre les infections. Ces peptides font l'objet d'un intérêt croissant, en particulier du fait qu'ils possèdent généralement un large spectre d'action, et une faible cytotoxicité pour les cellules eucaryotes.

Les comparaisons des séquences en acides aminés, des structures secondaires, et les similitudes fonctionnelle ont permis de définir quatre groupes principaux, auxquels on peut rattacher la plupart des peptides anti-microbiens décrits jusqu'à présent. Pour revue. cf. par exemple HOFFMANN et al., dans [Phylogenetic Perspectives In Immunity: The Insect Host Defense. Chapitre 4, pp. 43-65 (1994)]:
1. Un premier groupe comprend des peptides linéaires constitués essentiellement d'acides aminés basiques et hydrophobes ; dans ce groupe on classe en particulier les cécropines d'insectes et de mammifères, et les magainines de la peau des batraciens ;
2. Un deuxième groupe comprend des peptides comprenant des ponts disulfure intramoléculaires ; dans ce groupe, on range en particulier les défensines d'insectes ou de mammifères, les brévinines de la peau de batraciens, la thanatine d'insecte, qui présente une homologie importante de séquence avec les brévinines [FEHLBAUM et al. Proc. Natl. Acad. Sci. USA, 93, pp. 1221-1225,(1996)], les tachyplésines, produites par des arthropodes marins primitifs, ainsi que différents peptides obtenus à partir de l'hémolymphe de scorpion [EHRET-SABATIER et al., J. Biol. Chem., 271, 47, pp. 29537-29544, (1996)] ;
3. Le troisième groupe comprend des peptides riches en proline, parmi lesquels on peut classer les apidaecines et les abaecines des hyménoptères, la drosocine et la pyrrhocoricine, également produites par des insectes, et les bacténécines de mammifères. A cette classe appartient également le seul peptide anti-microbien produit par un crustacé décapode qui avait été caractérisé jusqu'à présent : il s'agit d'un peptide antibactérien riche en proline, similaire à la bacténécine-7, et obtenu à partir des hémocytes du crabe *Carcinus maenas* [SCHNAPP et al.. Eur.J.Biochem. 240, pp. 532-539 (1996)].
4. La quatrième classe comprend des peptides ou polypeptides riches en glycine, tels que les attacines, les sarcotoxines et les diptéricines, tous isolés chez les insectes.

Les Inventeurs ont maintenant purifié et caractérisé de nouveaux peptides anti-microbiens à partir de l'hémolymphe de crevettes pénéides, et ont également obtenu des séquences d'ADN codant pour ces peptides.

La présente invention a pour objet ces peptides anti-microbiens, dénommés ci-après penaeidines, qui possèdent les caractéristiques suivantes :
- leur masse moléculaire est d'environ 5 à 7 kDa ;
- leur pHi est supérieur ou égal à 9 ;
- leur séquence N-terminale comprend une région (A) de 15 à 25 acides aminés, riche en proline, (au moins 1/8, et de préférence entre 1/8 et 1/3 des acides aminés de cette région sont des prolines), et qui comprend la séquence (1) suivante :

   PX₁PX₂PX₃PX₁X₁X₂PX₄X₄ (I)

   dans laquelle P représente une proline, X₁ représente un acide aminé neutre non-polaire, X₂ représente un acide aminé basique, X₃représente une proline ou une liaison peptidique et X₄ représente un acide aminé neutre non-polaire ou une proline.
- leur portion C-terminale comprend une région (B) de 20 à 30 acides aminés, qui contient 6 résidus cystéine formant trois ponts disulfure intramoléculaires.

Selon un mode de réalisation préféré de la présente invention, la région (A) comprend la séquence (II) suivante :

PX₁PX₂PX₃PX₁X₁X₂PX₁PX₁X₁PX₁X₁P (II)

dans laquelle P, X₁, X₂ et X₃sont tels que définis ci-dessus.

Selon un autre mode de réalisation préféré de la présente invention, les 6 résidus cystéine de la région (B) sont disposés selon la séquence (III) suivante :

CS₁CS₂CCS₃CC (III)

dans laquelle C représente une cystéine, S₁ représente un acide aminé ou une séquence peptidique de 2 ou 3 acides aminés, S₂ représente une séquence peptidique de 10 acides aminés, S₃ représente une séquence peptidique de 5 acides aminés.

Selon un mode de réalisation préféré d'un peptide conforme à l'invention, son extrémité N-terminale est bloquée par un résidu d'acide pyroglutamique, et/ou son extrémité C-terminale est amidée.

A titre d'illustration de l'objet de la présente invention, les caractéristiques de 3 penaeidines isolées à partir de l'hémolymphe de la crevette *Penaeus vannamei*, dénommées ci-après penaeidine 1, penaeidine 2, et penaeidine 3, sont plus spécifiquement indiquées ci-dessous.

Les séquences de ces 3 peptides (code 1 lettre) sont représentées sur les figures 1a (penaeidine 1), 1b (penaeidine 2), et 1c (penaeidine 3) ; l'alignement des 3 séquences est représenté sur la figure 1d : les séquences conservées sont encadrées. La figure 2 représente une séquence d'ADNc de la penaeidine 2 et la séquence peptidique (code 3 lettres) correspondante; la figure 3A représente une séquence d'ADNc de la penaeidine 3 et la séquence peptidique (code 3 lettres) correspondante ; la figure 3B représente les séquences peptidiques (code 1 lettre) d'autres isoformes de la penaeidine 3 : les variations de séquence sont encadrées.

Les séquences d'ADNc de la penaeidine 2 et de la penaeidine 3 sont respectivement représentées dans la liste de séquences en annexe sous les numéros SEQ ID NO: 1 et SEQ ID NO: 3, et les séquences de leurs produits de traduction sont respectivement représentées dans la liste de séquences en annexe sous les numéros SEQ ID NO: 2 et SEQ ID NO: 4.

Les séquences peptidiques des formes matures des penaeidines 1, 2, et 3 sont respectivement représentées dans la liste de séquences en annexe sous les numéros SEQ ID NO: 5, SEQ ID NO: 6 et SEQ ID NO: 7.

Ces penaeidines ne présentent aucune homologie significative avec les peptides anti-microbiens connus dans l'art antérieur, et définissent un nouveau groupe de peptides anti-microbiens.

Les penaeidines 1 et 2 comprennent 50 acides aminés, et ont une masse moléculaire d'environ 5,5 kDa ; la penaeidine 3 comprend 62 acides aminés et sa masse moléculaire est d'environ 6,6 kDa.

Ce sont des peptides cationiques, possédant une charge nette positive de 7 pour les penaeidines 1 et 2, et de 8 pour la penaeidine 3 ; leurs points isoélectriques calculés varient de 9,34 pour les penaeidines 1 et 2, à 9,84 pour la penaeidine 3.

Les 3 peptides ont un domaine N-terminal riche en proline, et un domaine C-terminal comprenant 6 résidus cystéine formant trois ponts disulfure intramoléculaires, et où les 4 résidus cystéine les plus proches de l'extrémité C-terminale sont organisés en 2 doublets séparés par 5 résidus.

Les cystéines les plus centrales sont respectivement séparées par un, deux ou trois résidus dans les penaeidines 1, 2, et 3.

L'extrémité N-terminale de la penaeidine 3 est bloquée par un résidu d'acide pyroglutamique. Le blocage par des résidus similaires a déjà été observé chez d'autres peptides anti-microbiens.

L'extrémité C-terminale des penaeidines 2 et 3 est amidée. Une telle amidation C-terminale a déjà été observée pour certains peptides anti-microbiens d'autres invertébrés marins (tachyplésines de Limule), ainsi que pour les cécropines d'insectes et les magainines d'amphibiens. Une telle modification renforce la stabilité de la molécule, et il semble qu'elle augmente l'activité anti-microbienne.

Les penaeidines possèdent une stabilité élevée, et sont en particulier très résistantes à la protéolyse. Elles sont essentiellement actives contre les bactéries à Gram positif, et présentent également une activité contre les bactéries à Gram négatif ; elles possèdent en outre des propriétés fongicides.

Les penaeidines et leurs fragments, tels que définis ci-dessus, peuvent être obtenus par exemple par extraction à partir des animaux qui les produisent, et également par synthèse peptidique, ou avantageusement, par génie génétique, en exprimant au moins une séquence d'acide nucléique codant pour une penaeidine ou un fragment de celle-ci, dans une cellule hôte appropriée.

La présente Invention englobe également des acides nucléiques comprenant un segment du gène d'une penaeidine.

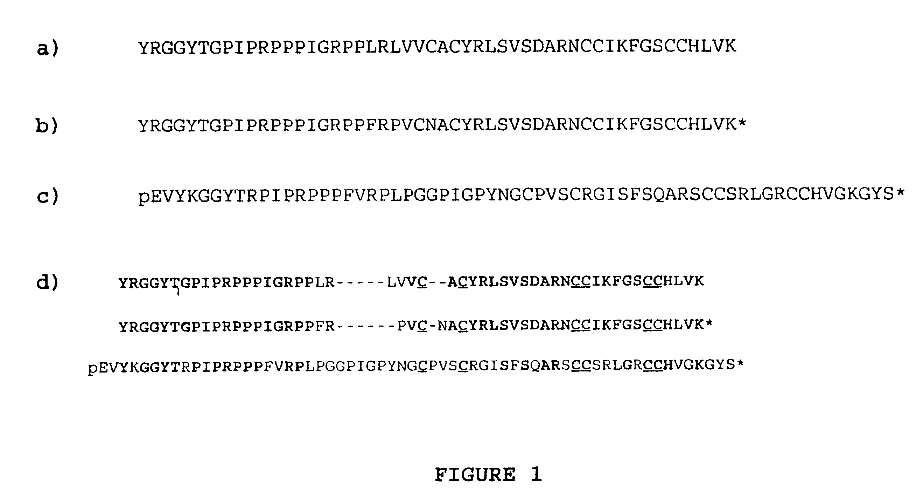

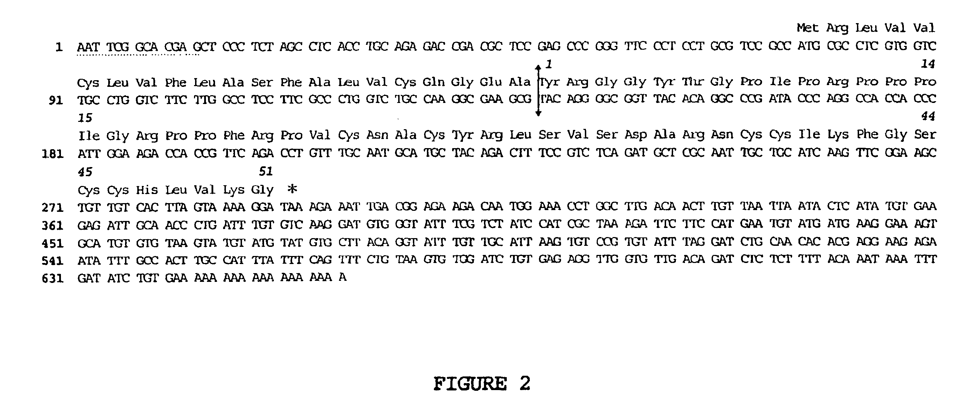

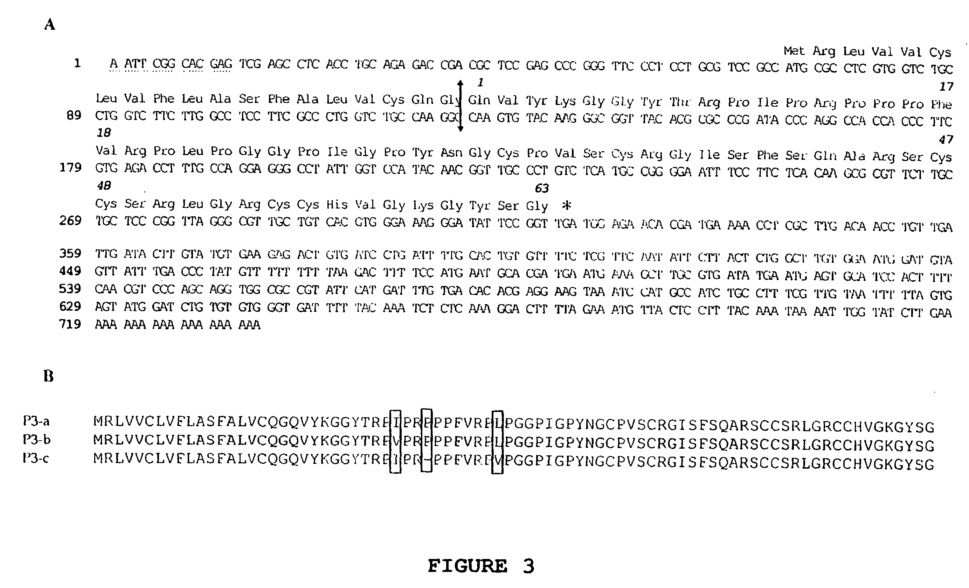

Des acides nucléiques conformes à l'invention, et en particulier des ADNc de penaeidine, ou des portions de ceux-ci peuvent être obtenus par criblage de banques d'acide nucléique à l'aide d'oligonucléotides dérivés des séquences représentées sur les figures 1, 2, ou 3, ou de leurs séquences complémentaires.

Des acides nucléiques conformes à l'invention englobent également des cassettes d'expression, comprenant au moins une séquence d'acide nucléique codant pour une penaeidine ou un fragment de celle-ci, sous contrôle transcriptionnel d'un promoteur approprié.

Par " promoteur approprié " on entend tout promoteur fonctionnel dans la cellule-hôte destinée à héberger la cassette d'expression.

Une cassette d'expression conforme à l'invention peut également comprendre en outre une ou plusieurs séquence(s) d'acide nucléique permettant d'améliorer la sécrétion de la penaeidine ou de son fragment par la cellule-hôte, par exemple une séquence codant pour un peptide signal. La séquence d'acide nucléique codant pour le peptide signal est placée à l'extrémité 5' de la séquence d'acide nucléique codant pour la penaeidine ou son fragment.

La séquence d'acide nucléique codant pour le peptide signal peut être une séquence codant pour un peptide signal de penaeidine, ou bien une séquence codant pour un peptide signal hétérologue. On choisira une séquence qui comprend à l'extrémité C-terminale un site de protéolyse capable d'être reconnu par une signal-peptidase de la cellule hôte destinée à héberger la cassette d'expression.

L'Invention a également pour objet :
- des vecteurs recombinants caractérisés en ce qu'ils comprennent au moins un acide nucléique conforme à l'invention, et en particulier des vecteurs comprenant une cassette d'expression telle que définie ci-dessus.
- des cellules procaryotes ou eucaryotes transformées par une cassette d'expression selon l'invention. Il peut s'agir de cellules maintenues en culture, ou de cellules faisant partie d'un organisme pluricellulaire, animal ou plante. La cassette d'expression présente dans la cellule transformée peut être soit incorporée dans l'ADN chromosomique de ladite cellule. soit être portée par un vecteur extra-chromosomique.

L'invention a également pour objet un procédé de production d'une penaeidine ou d'un fragment de celle-ci, caractérisé en ce qu'il comprend l'expression de ladite penaidine ou dudit fragment, dans au moins une cellule transformée conforme à l'invention.

Les peptides conformes à l'invention peuvent être exprimés dans des cultures de cellules transformées en utilisant des techniques similaires à celles utilisées pour des peptides antimicrobiens de l'art antérieur, par exemple dans des cellules d'insecte, comme décrit par HELLERS et al. [Eur. J. Biochem. 199, pp. 435-439, (1991)] pour les cécropines, ou dans la levure, comme décrit par REICHHART et al. [Invertebrate Reproduction and Development, 21, pp. 15-24, (1992)].

Ils peuvent également être exprimés dans des animaux ou des plantes transgéniques, pour augmenter la résistance de ceux-ci aux infections, comme décrit par exemple par JAYNES et al., [Plant Science, 89, pp. 43-53 (1993)] dans le cas de peptides analogues de la cécropine B, exprimés dans des plants de tabac transgénique, ou par NORELLI et al. [Euphytica, 77, pp. 123-128 (1994)] pour des plants de pommiers transgéniques exprimant le gène de l'attacine-E.

Les peptides conformes à l'invention sont utilisables en particulier pour l'obtention de produits et en particulier de médicaments anti-infectieux, par exemple anti-bactériens ou fongicides.

De tels produits trouvent leur application pour la prévention et le traitement de différentes maladies microbiennes, dans des secteurs très variés, en particulier dans les domaines de la santé et de l'agriculture, et dans celui de l'aquaculture, pour limiter le développement de maladies infectieuses dans les élevages.

La présente invention sera mieux comprise à l'aide du complément de description qui va suivre, qui se réfère à des exemples de purification des peptides anti-microbiens conformes à l'invention, et de leur caractérisation.

### EXEMPLE 1 : ISOLATION DE PEPTIDES ANTI-MICROBIENS A PARTIR DE LA CREVETTE PENAEUS VANNAMEI.

225 ml d'hémolymphe ont été obtenus à partir de 500 crevettes, par prélèvement à partir du sinus central. situé à la base du premier segment abdominal. Le prélèvement a été effectué en présence de tampon anticoagulant (10% citrate de sodium pH 7, additionné de 200 µM de phénylthiourée et 40 µg/ml d'aprotinine. L'hémolymphe a alors été centrifugée à 700g pendant 15 minutes à 4°C pour éliminer les cellules sanguines. Le plasma et les hémocytes sont conservés séparément à -70°C jusqu'à utilisation.

### 1.1 Préparation des fractions de l'hémolymphe par extraction acide

### a)Plasma :

Le plasma est dilué avec de l'eau déminéralisée (1 volume de plasma pour 1 volume d'eau), et à ce mélange sont ajoutés 0.1 % (v/v) d'acide trifluoroacétique. Le pH a été amené à 3,9 à l'aide HCl 1M dans un bain refroidi à la glace, sous agitation douce pendant 1 heure. Deux centrifugations successives à 8000g et à 4°C ont été effectuées afin de clarifier le surnageant qui est conservé sur la glace à 4° jusqu'à utilisation.

### b)Hémocytes :

### Cytosol

Après décongélation, les hémocytes sont homogénéisés à l'aide d'un appareil DOUNCE (maximum 152 µ, minimum 76 µ) dans du tampon Tris 50 mM pH 8,7 contenant 50 mM de NaCl. Après centrifugation (8000g 20 mn 4°C), le surnageant (fraction cytosol) est acidifié à un pH de 3,6 par addition de 1M HCl et conservé à 4°C.

### Organelles

Le culot contenant les organelles cellulaires est extrait par sonication (3 x 30 s) à puissance moyenne dans l'acide acétique 2 M. Les débris sont éliminés par centrifugation (8000g, 20 mn, 4°C) et l'extrait acide conservé à 4°C jusqu'à utilisation.

### 1.2 Purification des peptides.

### A)Extraction en phase solide :

La fraction plasmatique et les extraits cytosol et organelles ont été déposés séparément sur des cartouches SEP-PAK C₁₈ VAC de 35 cm3 (10 g WATERS ASSOCIATES), équilibrées avec de l'eau acidifiée (0,05% d'acide trifluoroacétique).

Après lavage à l'eau acidifiée, 3 élutions sont effectuées en utilisant successivement des solutions à 5%, 40%, et 80% d'acétonitrile dans l'eau acidifiée. Les différentes fractions obtenues sont lyophilisées, et reconstituées avec de l'eau microfiltrée préalablement à la purification par HPLC en phase inverse.

### B)Purification HPLC

### 1 : HPLC en phase inverse.

Les fractions éluées à 5% et 40% de la colonne SEP-PAK ont été soumises à une chromatographie en phase inverse sur une colonne AQUAPORE RP300 C₈ (4,6 x 220 mm, BROWNLEE™), équilibrée dans l'eau acidifiée (0,05% TFA). La séparation de la fraction 5% SEP-PAK est effectuée à l'aide d'un gradient linéaire de 0 à 5% d'acétonitrile dans l'eau acidifiée, à un débit de 1 ml/mn pendant 80 mn.

Pour la fraction 40%, un gradient linéaire de 2 à 60% d'acétonitrile est utilisé dans les mêmes conditions. Les fractions sont collectées, séchées sous vide, reconstituées à l'aide d'eau ultrafiltrée, et leur activité anti-microbienne est testée.

### 2 : Chromatographie d'exclusion.

Les fractions obtenues à l'issue de la chromatographie en phase inverse présentant une activité anti-microbienne sont purifiées par chromatographie d'exclusion, en utilisant 2 colonnes HPLC reliées en série (colonne ULTRASPHEROGEL SEC 3000 et colonne ULTRASPHEROGEL SEC 2000, 7,5 × 300 mm, BECKMAN) protégées par une précolonne (ULTRASPHEROGEL SEC, 7,5 × 40 mm, BECKMAN).

L'élution est effectuée en condition isocratiques en présence de 30% d'acétonitrile dans l'eau acidifiée, à un débit de 0.5 ml/mn. Les fractions sont collectées et testées pour leur activité anti-microbiennes.

### 3 : Chromatosraphie en phase inverse.

Les peptides ont été purifiés par une chromatographie en phase inverse sur la même colonne que dans l'étape 1 à une température contrôlée de 35°C.

Les peptides 1 et 2 ont été purifiés avec un gradient biphasique linéaire : de 2 à 21% d'acétonitrile dans l'eau acidifiée (0,05% TFA) pendant 10 mn, puis de 21 à 35% d'acétonitrile pendant 50 mn à un débit de 0,25 ml/mn.

Le peptide 3 a été purifié à l'aide d'un gradient biphasique linéaire : de 2 à 23 % d'acétonitrile dans l'eau acidifiée pendant 10 mn, puis de 23 à 37% d'acétonitrile pendant 50 mn à un débit de 0,25 ml/mn à 35°C.

### 4 : Dernières étapes de purification.

Les dernières étapes de purification des peptides ont été effectuées sur colonne phase-inverse DELTA PAK HPI C₁₈, 2 x 150 mm, WATERS ASSOCIATES, à une température de 40°C avec un débit de 0,25 ml/mn en utilisant les gradients biphasiques décrits ci-dessus.

L'élution est contrôlée par absorption dans l'ultra-violet à 225 nm. Les fractions sont collectées dans des tubes en polypropylène, concentrées sous vide et reconstituées avec de l'eau stérilisée par filtration préalablement à la mesure de l'activité anti-microbienne.

### EXEMPLE 2: ACTIVITE ANTIMICROBIENNE DES FRACTIONS OBTENUES

Les fractions 40% SEP PAK obtenues à partir de l'extrait plasmatique, à partir des organelles des hémocytes, et à partir de la fraction cytosolique, qui sont respectivement désignées ci-après sous les dénominations P40, HO40 et HC40, ont été principalement étudiées. Toutes les fractions ont été testées pour leur activité contre 2 souches bactériennes et contre un champignon filamenteux.

### Micro-organismes :

Les souches microbiennes utilisées pour déterminer les activités anti-microbiennes sont les suivantes :
- *Micrococcus luteus* (souche à Gram positif) ;
- *Escherichia coli* (souche à Gram négatif) ;
- *Neurospora crassa* (champignon filamenteux) ;

Les bactéries sont cultivées avec une densité optique de départ A₆₀₀ = 0,001 en milieu nutritif « Poor-Broth » (1% bactotryptone. 0,5% NaCl p/v).

### Essais antibactériens :

Les fractions ont été testées par inhibition de la croissance en phase liquide.

Des aliquotes de 10 µl de chaque fraction à tester sont incubés dans des plaques de micro-titration avec 100 µl d'une suspension de bactéries en milieu de phase logarithmique.

La croissance bactérienne est mesurée en déterminant la densité optique A₆₀₀ après une incubation de 24 heures à 30°C.

Une procédure identique a été utilisée pour déterminer la concentration minimale inhibitrice (CMI) des molécules sur ces souches bactériennes. Les valeurs de la CMI correspondent à un intervalle a-b de concentrations en peptide, où a représente la concentration la plus élevée à laquelle on observe une croissance bactérienne, et b est la concentration la plus faible qui cause 100% d'inhibition de croissance.

### Mesure des propriétés bactériostatiques :

Une culture de *Micrococcus luteus* en milieu de phase logarithmique, dans le milieu « Poor-Broth » défini ci-dessus, a été incubée à 30°C en présence du peptide ou (à titre de témoin) d'eau. La concentration finale des molécules testées est 8 fois supérieure à la CMI. Des aliquotes de 20 µl sont prélevées à divers intervalles de temps et mises en culture sur plaque d'agar nutritif. Le nombre de colonies formées (UFC) est déterminé après 24 heures d'incubation à 37°C.

### Activité antifongique :

L'activité antifongique a été déterminée contre *Neurospora crassa* et *Fusarium oxysporum* par un test d'inhibition de croissance en phase liquide.

80 µl de spores de champignons (concentration finale : 10⁴ spores/ml) sont suspendus dans du 1/2 Potato Dextrose Broth (DIFCO) supplémenté avec de la tétracycline (10 µg/ml) et de la céfotaxime (100 µg/ml). Cette suspension a été additionnée à 10 µl de la fraction à tester dans des plaques de microtritation. Le volume final est amené à 100 µl par addition de 10 µl d'eau. L'inhibition de la croissance peut être observée au microscope après 24 heures d'incubation à 25°C dans l'obscurité, et mesurée par la variation de densité optique à 600 nm au bout de 48 heures.

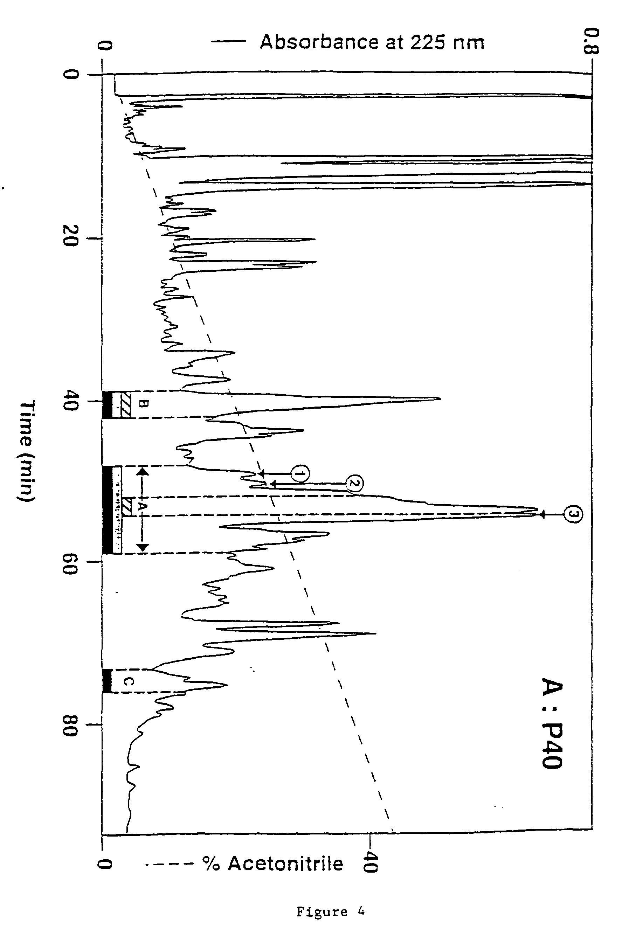

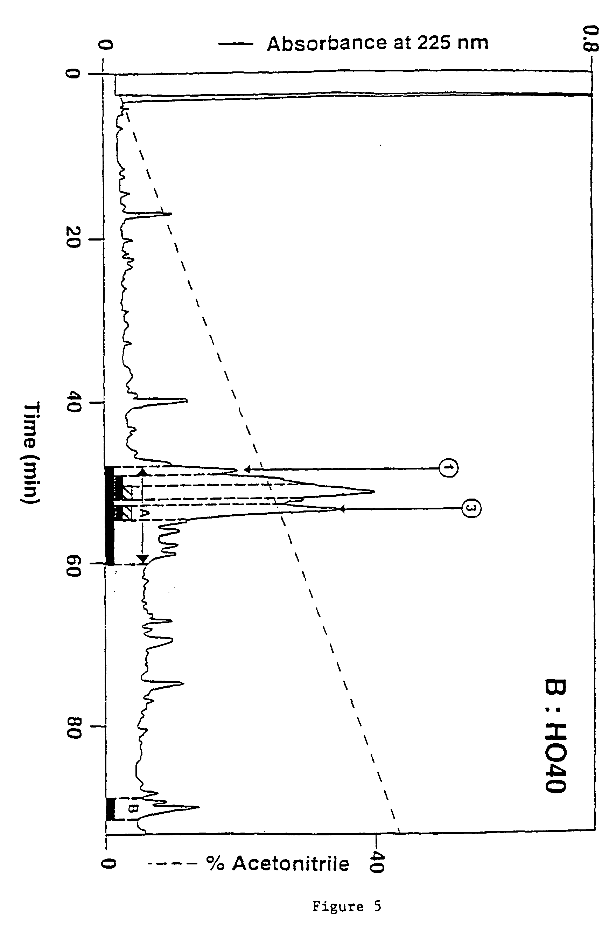

Aucune activité n'a été obtenue pour les fractions obtenues à partir de HC40. En revanche, comme le montrent respectivement les figures 4 et 5, certaines des fractions obtenues à partir de P40 et de HO40 présentent une activité anti-microbienne, et possèdent une zone active commune, correspondant aux fractions éluées entre 47 et 60 mn (26 à 29% d'acétonitrile), où l'on observe une activité contre les 2 souches bactériennes et le champignon.

### Légende des figures 4 et 5 :

Le temps d'élution en mn est indiqué en abscisse. L'absorbance à 225 nm est représentée en ordonnée. L'activité anti-microbienne contre *Escherichia coli* D31 (rectangle hachuré), *Micrococcus luteus* (rectangle noir) et *N*.*crassa* (rectangle gris) est indiquée pour les fractions concernées. Les régions de la courbe correspondant aux fractions présentant une activité anti-microbienne sont indiquées par les lettres A, B et C. Les fractions à partir desquelles ont été purifiés les penaeidines 1, 2 et 3 sont indiquées par les flèches 1, 2 et 3.

2 régions présentant également une activité anti-microbienne ont été obtenues après chromatographie en phase inverse de la fraction plasmatique. Les fractions P40B, qui sont éluées vers 40 mn (21-22% d'acétonitrile), contiennent des molécules actives contre les 3 micro-organismes testés, et P40C, correspondant à des molécules éluées à environ 75 mn, 38-39% d'acétonitrile, sont actives uniquement contre *Micrococcus luteus*. Aucune de ces deux zones n'est présente dans le chromatogramme de HO40, dans lequel d'autres fractions éluées à environ 90 mn (45-46% d'acétonitrile) sont actives seulement contre *Micrococcus luteus*.

### EXEMPLE 3 : DETERMINATION DE LA STRUCTURE DES PEPTIDES OBTENUS.

Les trois peptides purifiés comme indiqué à l'exemple 1 ci-dessus ont été caractérisés par des techniques successives de biochimie et de biologie moléculaire.

Leur masse moléculaire a été déterminée par spectrométrie de masse ; cette masse moléculaire est respectivement de 5484,8 Da et 5520,0 Da pour les peptides P1 et P2, et de 6617,4 Da pour le peptide P3.
* La séquence d'acides aminés de P1 a pu être obtenue par séquencage direct par dégradation d'Edman.
   P1 est un peptide de 50 résidus, contenant 14% de proline parmi les 19 résidus N-terminaux, et 6 cystéines formant 3 ponts disulfure intramoléculaires dans le domaine C-terminal. P1 est riche en acides aminés basiques, comme l'indique la présence de 5 arginines et 2 lysines distribuées sur toute la longueur du peptide.
* En ce qui concerne le peptide P2, le séquençage direct n'a permis d'obtenir qu'une séquence partielle de 21 résidus N-terminaux. Cette séquence ne diffère de celle de la penaeidine 1 que par le remplacement de la leucine en position 20 chez P1 par une phénylalanine chez P2.
* En ce qui concerne le peptide P3, sa séquence N-terminale n'a pas pu être établie par la technique de dégradation d'Edman, ce qui suggère un blocage à son extrémité N-terminale.

La S-pyridyléthylation suivie par l'analyse en spectrométrie de masse, et la détection de la variation de masse induite par la S-pyridyléthylation. montre la présence de 6 résidus cystéines. Après clivage enzymatique du peptide S-pyridyléthylé, les fragments obtenus ont été purifiés, analysés par spectrométrie de masse, et par dégradation d'Edman. La séquence de l'extrémité NH₂-terminale a été déterminée par nanoES-MS (nano Electrospray Mass Spectrometry). Il a ainsi pu être déterminé que le résidu NH₂-terminal de ce peptide était cyclisé en acide pyroglutamique.

### EXEMPLE 4 : CLONAGE DES ADN_{c} DES PENAEIDINES.

### - ADNc de la penaeidine 3 :

Une sonde constituée d'oligonucléotides dégénérés correspondant aux résidus 38-44 du peptide P3, et présentant la séquence suivante : a été préparée.

Cette sonde a permis d'obtenir par transcription inverse suivie d'amplification en chaîne par polymérase (PCR) à partir des ARN poly(A)⁺ totaux d'hémocytes de crevettes adultes. récoltés 6 et 12 heures après un challenge bactérien, un produit d'amplification constitué par un fragment de 497 paires de bases. Après séquençage, ce fragment a été identifié comme un fragment de l'ADNc de P3, constitué par l'extrémité du cadre de lecture ouvert et la région 3' non traduite. Un sous-fragment de 440 paires de bases, obtenu par digestion enzymatique BsaI à partir de ce fragment de 497 paires de bases, et constitué principalement de la région 3' non-traduite, a été cloné dans un vecteur pBluescript (STRATAGENE, La Jolla, CA). Ce fragment a été marqué par amorçage aléatoire en utilisant le kit de marquage d'ADN READY-TO-GO (PHARMACIA BIOTECH, Uppsala, Sweden), et utilisé pour cribler une banque d'ADNc obtenus à partir des ARN poly(A)± de crevettes adultes, dans le vecteur ZAP-EXPRESS (STRATAGENE, La Jolla, CA).

Des hybridations à stringence élevée ont été effectuées pendant une nuit à 65°C dans une solution de Denhardt 5X, 5X SSPE, 0.1% SDS, en présence de 100 µg/µl d'ADN de sperme de saumon.

161 clones positifs ont été obtenus. Parmi ces clones, 4 ont été séquencés. L'un d'entre eux contenait un cadre de lecture ouverte codant pour une séquence de 81 acides aminés (P3a) débutant par un codon méthionine et se terminant par un codon stop.

La séquence en acides aminés déduite de ce cadre de lecture ouverte commence par un peptide signal de 19 résidus riche en résidus hydrophobes. Le site de clivage de la signal-peptidase a été localisé après le résidu glycine précédant la glutamine en position 1.

Ce peptide-signal est directement suivi. à son extrémité C-terminale, par un peptide de 63 acides aminés, débutant par un résidu glutamine. La séquence en acides aminés déduite de ce peptide mature confirme clairement les séquences partielles de la penaeidine 3 obtenues par séquençage direct.

En partant de l'hypothèse que la penaeidine 3 sous forme mature débute par un acide pyroglutamique résultant de la cyclisation du résidu glutamine (observée par approche biochimique), la masse calculée à partir de la séquence en acides aminés déduite est supérieure de 56.4 Da à la masse mesurée. Ceci suggère que la penaeidine 3 est amidée à son extrémité C-terminale par élimination d'un résidu glycine (57 Da).

Parmi les 3 autres clones séquences, 2 séquences déduites en acides aminés légèrement différentes ont été identifiées (P3b etP3c). P3b diffère de P3a par le remplacement d'une isoleucine à la position 30 chez P3a par une valine chez P3b, alors que P3c est dépourvu, en position 33, d'une proline qui est présente chez P3a et P3b. Enfin, la leucine en position 40 chez P3a et P3b est remplacée par une valine chez P3c.

### - ADNc de la penaeidine 2 :

Afin d'isoler les ADNc des autres penaeidines, une sonde correspondant à une portion de la séquence codante de la penaeidine 3 dont la séquence du produit de traduction est fortement conservée entre les 3 peptides purifiés, a été utilisée.

Cette sonde a été obtenue par amplification en chaîne par polymérase sur un clone d'ADNc de P3, en utilisant les amorces suivantes :
amorce amont : 5'GTGTACAAGGGCGGTTACACG3' (SEQ ID NO: 8).
amorce aval : 5'CAACAGGTTGTCAAGCGAGGT3' (SEQ ID NO: 9).

L'hybridation est effectuée dans les mêmes conditions que celles décrites ci-dessus, mais à une température d'hybridation plus faible (50°C), afin d'assurer une stringence moins élevée.

Les clones obtenus dans ces conditions ont été analysés sur la base de leur profil de restriction. Pour l'un de ces clones, ce profil était différent de celui observé pour P3a. Ce clone a été séquencé. La séquence en acides aminés déduite de la séquence d'ADN possède un pourcentage d'identité très élevé avec la penaeidine 1, en particulier un domaine COOH-terminal totalement identique à celui établi par séquençage direct de la penaeidine 1. Cependant, la présence d'un résidu phénylalanine en position 20 dans le peptide mature, ainsi que la valeur de la masse moléculaire, est en faveur de l'ADNc de la penaeidine 2. La séquence en acides aminés déduite comporte un acide aminé supplémentaire (glycine à l'extrémité C-terminale, par rapport à celle obtenue par séquençage direct, ce qui suggère que ce résidu glycine peut être éliminé par amidation. Ceci a été confirmé par le calcul de la masse (5575,6 Da) qui est supérieure de 55,6 Da à la masse mesurée pour la penaeidine 2, alors que la masse calculée du peptide amidé correspond à celle de la penaeidine 2 mesurée par spectrométrie de masse.

Il apparait en outre que la penaeidine 2 provient d'une molécule précurseur possédant une région « pré » de 21 résidus, identique à celle du peptide-signal de la penaeidine 3, avec deux résidus supplémentaires (Glu-Ala) précédant immédiatement le site de clivage observé. Un autre site de clivage potentiel a été localisé à la position -3 en amont du résidu Tyr-1. Cet autre site correspond à celui qui est observé dans la maturation de la penaeidine 3.

### EXEMPLE 5 : ACTIVITE ANTI-MICROBIENNE DE LA PENAEIDINE 3 PURIFIEE.

L'activité de la penaeidine 3 purifiée a été testée comme décrit à l'exemple 2, contre *Micrococcus luteus*, *Escherichia coli* D31, *Neurospora crassa*, et également contre *Fusarium oxysporum* qui est un champignon pathogène des crevettes pénéides.

Ce peptide a une activité marquée contre *Micrococcus luteus* (CMI = 0,6 à 2,5 µM) et une activité modérée sur *Escherichia coli* (CMI>5 µM). La penaeidine 3 est également active contre les 2 champignons testés (CMI>5 µM).

Lorsque l'on procède à l'incubation de la penaeidine 3 avec *Micrococcus luteus* à une concentration de 18 µM, c'est-à-dire 8 fois supérieure à la CMI, aucune croissance bactérienne n'est observée après une incubation de 24 heures. De plus, le nombre de colonies formées reste constant au cours des différents prélèvements, ce qui suggère que cette molécule a un effet bactériostatique. Ces résultats sont illustrés par le tableau 2 ci-dessous.

**TABLEAU II**

| **Temps d'incubation** | **Contrôle** | **Penaeidine 3** |
|---|---|---|
| *10*^{*4*} *ufc*/*ml* | | |
| 1 min | 3.85 | 4,30 |
| 30 min | 4,40 | 4,45 |
| 2 h | 6.70 | 3,15 |
| 4 h | 9,45 | 4,60 |
| 7 h | 51,50 | 4,50 |
| 24 h | >1 000 | 5,60 |

### EXEMPLE 6 : PRODUCTION DE PENAEIDINES RECOMBINANTES

Des fragments d'ADN codant pour les pénaeidines-2 ou -3 flanquées à leur extrémité amino-terminale ont été obtenus par PCR en utilisant les amorces suivantes. qui permettent d'insérer, en amont de la séquence codant pour la pénaeidine, une séquence codant pour les cinq derniers résidus de la prorégion du facteur sexuel MFα1 de levure et un site HindIII, et en aval de la séquence codant pour la pénaeidine, un site BamHI.
Amorces utilisées pour la pénaeidine-2 : Ces 2 amorces sont respectivement représentées dans la liste de séquences sous les numéros SEQ ID NO: 10 et SEQ ID NO: 11
Amorces utilisées pour la pénaeidine-3 : Ces 2 amorces sont respectivement représentées dans la liste de séquences sous les numéros SEQ ID NO: 12 et SEQ ID NO: 13

L'enzyme utilisée pour la PCR est la Vent polymérase (NEW ENGLAND BIOLABS) qui génère des extrémités franches. Les fragments obtenus ont été clonés entre les sites HindIII et BamHI du lieur multisite du plasmide pCR-SCRIPT (STRATAGENE).

Les inserts des plasmides ainsi obtenus ont été excisés par BamHI et HindIII, et sous-clonés dans la cassette d'expression de levure du vecteur pJV1. Le vecteur pJV a été fourni par le laboratoire du Professeur HOFFMANN à Strasbourg : il s'agit d'un plasmide dérivé de pBLUESCRIPT II dans lequel ont été introduits le promoteur du facteur sexuel MFα1 de levure et la préproséquence de ce même facteur, dans laquelle on a créé par mutation silencieuse un site de restriction HindIII permettant la fusion en phase des séquences codantes.

Les cassettes complètes (environ 1,4 kb) ont été excisées de pJV1 par double digestion SphI-BamHI (digestion Sphl partielle pour la pénaeidine-2) et sous-clonées dans le vecteur navette pTG4812 [MICHAUD et al. FEBS Lett. **395**: 6-10 (1996)].

Les deux vecteurs d'expression ainsi obtenus sont dénommés pen2-pTG4812 et pen3-pTG4812, et permettent respectivement l'expression de la pénaeidine 2 et celle de la pénaeidine 3 chez la levure.

Pour la production des pénaeidines recombinantes, on a utilisé la souche de *Saccharomyces cerevisiae* TGY48-1, décrite par REICHHART et al. [Invert. Reprod. Dev. **21**(1): 15-24 (1992). Elle porte une mutation *ura3-Δ5* qui permet la sélection de clones recombinants sur milieu sélectif dépourvu d'uracile.

Cette souche a été transformée avec les plasmides recombinants pen2-pTG4812 et pen3-pTG4812selon la méthode à l'acétate de lithium décrite par GIETZ et al. [Nucl. Ac. Res. **20**(6): 1425 (1992)]. Les clones de levure recombinants ont été sélectionnés sur milieu sélectif « YNBG-casaminoacids dépourvu d'uracile, et mis en culture. Les pénaeidines recombinantes sont sécrétées dans le milieu de culture, et sont purifiées à partir du surnageant de culture selon un protocole similaire à celui décrit dans l'exemple 1 ci-dessus.

## Revendications

1. Peptide anti-microbien, **caractérisé en ce qu'**il est susceptible d'être obtenu à partir de crevettes pénéides, et **en ce que**
- sa masse moléculaire est d'environ 5 à 7 kDa ;
- son pHi est supérieur ou égal à 9 ;
- sa portion N-terminale comprend une région (A) de 15 à 25 acides aminés, qui comprend la séquence (I) suivante :
PX₁PX₂PX₃PX₁X₁X₂PX₄X₄ (I)
dans laquelle P représente une proline, X₁ représente un acide aminé neutre non-polaire, X₂ représente un acide aminé basique, X₃ représente une proline ou une liaison peptidique et X₄ représente un acide aminé neutre non-polaire ou une proline;
- sa portion C-terminale comprend une région (B) de 20 à 30 acides aminés, qui contient 6 résidus cystéine formant trois ponts disulfure intramoléculaires.

2. Peptide selon la revendication 1, **caractérisé en ce que** la région (A) comprend la séquence (II) suivante :
PX₁PX₂PX₃PX₁X₁X₂PX₁PX₁X₁PX₁X₁P (II)
dans laquelle P, X₁, X₂et X₃sont tels que définis ci-dessus.

3. Peptide selon une quelconque des revendications 1 à 2, **caractérisé en ce que** les 6 résidus cystéine de la région (B) sont disposés selon la séquence (III) suivante :
CS₁CS₂CCS₃CC (III)
dans laquelle C représente une cystéine, S₁ représente un acide aminé ou une séquence peptidique de 2 ou 3 acides aminés, S₂ représente une séquence peptidique de 10 acides aminés, S₃ représente une séquence peptidique de 5 acides aminés.

4. Peptide selon une quelconque des revendications 1 ou 3 choisi dans le groupe constitué par :
le peptide de séquence SEQ ID NO: 5
le peptide de séquence SEQ ID NO: 6
le peptide de séquence SEQ ID NO: 7.

5. Peptide selon l'une quelconque des revendications 1 à 4, **caractérisé en ce que** son extrémité N-terminale est bloquée par un résidu d'acide pyroglutamique, et/ou son extrémité C-terminale est amidifiée.

6. Acide nucléique comprenant une séquence codant pour un peptide selon une quelconque des revendications 1 à 5.

7. Cassette d'expression, comprenant au moins une séquence d'acide nucléique selon la revendication 6, sous contrôle transcriptionnel d'un promoteur approprié.

8. Vecteur recombinant **caractérisé en ce qu'**il comprend au moins un acide nucléique selon la revendication 6.

9. Cellule procaryote ou eucaryote transformée par une cassette d'expression selon la revendication 7.

10. Procédé de production d'un peptide selon l'une quelconque des revendications 1 à 5, **caractérisé en ce qu'**il comprend l'expression d'un acide nucléique selon la revendication 6, dans au moins une cellule transformée selon la revendication 9.

11. Utilisation d'un peptide selon l'une quelconque des revendications 1 à 5 pour l'obtention d'un médicament.

## Patentansprüche

1. Antimikrobielles Peptid, **dadurch gekennzeichnet, dass** es geeignet ist, aus peneiden Krustentieren erhalten zu werden, und dass
- seine Molekularmasse ungefähr 5 bis 7 kDa beträgt;
- sein pHᵢ-Wert größer oder gleich 9 ist;
- sein N-terminaler Abschnitt eine Region (A) von 15 bis 25 Aminosäuren umfasst, welche die folgende Sequenz (I) umfasst:
PX₁PX₂PX₃PX₁X₁X₂PX₄X₄ (I)
in der P Prolin darstellt, X₁ eine neutrale unpolare Aminosäure darstellt, X₂ eine basische Aminosäure darstellt, X₃ Prolin oder eine peptidische Bindung darstellt und X₄ eine neutrale, nicht polare Aminosäuren oder Prolin repräsentiert;
- sein C-terminaler Abschnitt eine Region (B) von 20 bis 30 Aminosäuren umfasst, die 6 Cysteinreste enthalten, welche 3 intramolekulare DisulfidBrücken ausbilden.

2. Peptid gemäß Anspruch 1, **dadurch gekennzeichnet, dass** die Region (A) die folgende Sequenz (II) umfasst:
PX₁PX₂PX₃PX₁X₁X₂PX₁PX₁X₁PX₁X₁P (II)
in denen P, X₁, X₂ und X₃ den oben genannten Definitionen entsprechen.

3. Peptid gemäß irgendeinem der Ansprüche 1 bis 2, **dadurch gekennzeichnet, dass** die 6 Cysteinreste der Region (B) gemäß der folgenden Sequenz (III) angeordnet sind:
CS₁CS₂CCS₃CC (III)
in der C Cystein repräsentiert, S₁ eine Aminosäure oder eine Peptidsequenz aus 2 oder 3 Aminosäuren darstellt, S₂ eine Peptidsequenz aus 10 Aminosäuren darstellt, S₃ eine Peptidsequenz aus 5 Aminosäuren darstellt.

4. Peptid gemäß irgendeinem der Ansprüche 1 oder 3 ausgewählt aus der Gruppe bestehend aus:
dem Peptid der Sequenz SEQ ID NO: 5
dem Peptid der Sequenz SEQ ID NO: 6
dem Peptid der Sequenz SEQ ID NO: 7

5. Peptid gemäß irgendeinem der Ansprüche 1 bis 4, **dadurch gekennzeichnet, dass** sein N-terminales Ende durch einen Pyroglutaminsäure-Rest blockiert ist und/oder sein C-terminales Ende mit einer Amidgruppe versehen ist.

6. Nukleinsäure umfassend eine Sequenz, welche ein Peptid gemäß irgendeinem der Ansprüche 1 bis 5 kodiert.

7. Expressionskassette umfassend mindestens eine Nukleinsäuresequenz gemäß Anspruch 6, unter der Transkriptionskontrolle eines angepassten Promotors.

8. Rekombinanter Vektor, **dadurch gekennzeichnet, dass** er mindestens eine Nukleinsäure gemäß Anspruch 6 umfasst.

9. Prokaryotische oder eukariotische Zelle, welche mit einer Expressionskassette gemäß Anspruch 7 transformiert ist.

10. Verfahren zur Herstellung eines Peptides gemäß irgendeinem der Ansprüche 1 bis 5, **dadurch gekennzeichnet, dass** es die Expression einer Nukleinsäure gemäß Anspruch 6 in mindestens einer gemäß Anspruch 9 transformierten Zelle umfasst.

11. Verwendung eines Peptides gemäß irgendeinem der Ansprüche 1 bis 5, um ein Medikament zu erhalten.

## Claims

1. Antimicrobial peptide, **characterized in that** it can be obtained from penaeid prawns, and **in that**
- its molecular mass is about 5 to 7 kDa;
- its pHi is greater than or equal to 9;
- its N-terminal portion comprises a region (A) of 15 to 25 amino acids, which comprises the following sequence (I):
PX₁PX₂PX₃PX₁X₁X₂PX₄X₄ (I)
in which P represents a proline, X₁ represents a nonpolar neutral amino acid, X₂ represents a basic amino acid, X₃ represents a proline or a peptide bond and X₄ represents a nonpolar neutral amino acid or a proline;
- its C-terminal portion comprises a region (B) of 20 to 30 amino acids, which contains 6 cysteine residues forming three intramolecular disulphide bridges.

2. Peptide according to Claim 1, **characterized in that** region (A) comprises the following sequence (II):
PX₁PX₂PX₃PX₁X₁X₂PX₁PX₁X₁PX₁X₁P (II)
in which P, X₁, X₂ and X₃ are as defined above.

3. Peptide according to any one of Claims 1 to 2, **characterized in that** the 6 cysteine residues of region (B) are arranged according to the following sequence (III):
CS₁CS₂CCS₃CC (III)
in which C represents a cysteine, S₁ represents an amino acid or a peptide sequence of 2 or 3 amino acids, S₂ represents a peptide sequence of 10 amino acids, S₃ represents a peptide sequence of 5 amino acids.

4. Peptide according to any one of Claims 1 or 3, chosen from the group consisting of:
the peptide having the sequence SEQ ID NO: 5
the peptide having the sequence SEQ ID NO: 6
the peptide having the sequence SEQ ID NO: 7.

5. Peptide according to any one of Claims 1 to 4, **characterized in that** its N-terminal end is blocked by a pyroglutamic acid residue, and/or its C-terminal end is amidated.

6. Nucleic acid comprising a sequence encoding a peptide according to any one of Claims 1 to 5.

7. Expression cassette, comprising at least one nucleic acid sequence according to Claim 6, under transcriptional control of an appropriate promoter.

8. Recombinant vector, **characterized in that** it comprises at least one nucleic acid according to Claim 6.

9. Prokaryotic or eukaryotic cell transformed with an expression cassette according to Claim 7.

10. Method of producing a peptide according to any one of Claims 1 to 5, **characterized in that** it comprises the expression of a nucleic acid according to Claim 6, in at least one transformed cell according to Claim 9.

11. Use of a peptide according to any one of Claims 1 to 5 for the production of a medicament.
